# EUROPÄISCHE PATENTSCHRIFT

(11) **EP 4 370 060 B1**
(45) Veröffentlichungstag und Bekanntmachung des Hinweises auf die Patenterteilung: **18.03.2026**
(21) Anmeldenummer: 22753612.5
(22) Anmeldetag: 14.07.2022
(51) Int. Cl.: A61C 19/045, A61B 5/11

(54) **VERFAHREN ZUR ANIMIERTEN BILDLICHEN DARSTELLUNG VON BEWEGUNGEN DER ZÄHNE DES UNTERKIEFERS SOWIE SYSTEM ZU DESSEN DURCHFÜHRUNG**
METHOD FOR THE ANIMATED PICTORIAL REPRESENTATION OF MOVEMENTS OF THE TEETH OF THE LOWER JAW, AND SYSTEM FOR THE IMPLEMENTATION THEREOF
PROCÉDÉ DE REPRÉSENTATION GRAPHIQUE ANIMÉE DE MOUVEMENTS DES DENTS DE LA MÂCHOIRE INFÉRIEURE ET SYSTÈME POUR SA MISE EN OEUVRE

(30) Priorität: 14.07.2021 DE 102021118139
(43) Veröffentlichungstag der Anmeldung: 22.05.2024
(73) Patentinhaber: zebris Medical GmbH, 88316 Isny im Allgäu (DE)
(72) Erfinder: BRUNNER, Wolfgang, 88316 Isny (DE)
(74) Vertreter: Meissner Bolte Partnerschaft mbB
(86) Internationale Anmeldenummer: PCT/EP2022/069732
(87) Internationale Veröffentlichungsnummer: WO 2023/285594

(56) Entgegenhaltungen:
- CN-A- 112 790 888
- DE-A1- 102018 126 097
- JP-A- 2020 501 830

## Beschreibung

Die Erfindung betrifft ein Verfahren zur animierten bildlichen Darstellung von Bewegungen der Zähne des Unterkiefers, welches letztlich zur Erfassung der Bisslage und/oder von Bewegungsparametern anatomischer Punkte des Unterkiefers und/oder der Kieferrelation und/oder der Okklusion bei einem Wirbeltier, insbesondere dem Menschen, dient. Sie betrifft des Weiteren ein System zur Durchführung dieses Verfahrens.

Zur Durchführung zahnärztlicher Restaurationen und der Herstellung von Zahnersatz und Aufbissbehelfen verwendet man mechanische und neuerdings auch virtuelle Artikulatoren. Dazu muss die Bisslage des Patienten bestimmt werden. Hierzu verwendet man traditionell einen mechanischen Gesichtsbogen mit einer in den Mund geführten Bissplatte und misst deren Relation zum Gehörgang (steht in Relation zu den Kondylen) und dem Nasion (oder Orbitalpunkt) im Gesichtsbereich. Die gemessene Bisslage wird dann mittels OK-Gipsmodell in den mechanischen Artikulator übertragen.

Im Weiteren ist es notwendig, Bewegungsparameter anatomischer Punkte des Unterkiefers, insbesondere die Bewegungscharakteristika des Kondylen (links und rechts) sowie die der Frontzähne, zu bestimmen und die Kennzahlen der Bewegungsbahnen am Artikulator einzustellen.

Im Weiteren kann es wichtig sein, eine neue therapeutische Kieferrelation bzw. einen richtigen Aufbiss zwischen den Zähnen des Oberkiefers-OK und des Unterkiefers zu bestimmen. Ein vorhandener habitueller Aufbiss - mit ggf. abradierten Zähnen - kann sich ästhetisch und funktionell negativ auswirken und auch eine CMD (Cranio Manibuläre Dysfunktion) auslösen.

Im Weiteren ist es interessant, Informationen über die Okklusion zu erhalten. Dazu wird traditionell eine abfärbende Okklusionsfolie eingesetzt. Eine stimmige Okklusion - auch in der Bewegung - erhöht den Tragekomfort und die Haltbarkeit und Lebensdauer von Zahnersatz. Moderne Keramikmaterialien schleifen sich nicht mehr ein, sondern neigen dazu, zu brechen und/oder gesunde Antagonisten zu beschädigen.

DE 102 18 435 A1 beschreibt ein Verfahren und eine Vorrichtung zur 3-dimensionalen Bewegungsanalyse von Zahnoberflächen des Unterkiefers in Relation zum Oberkiefer. Es wird eine Sensorhalterung für den Unterkiefer gezeigt, von der ein Positionssensor gehalten werden kann.

Konkret wird in der DE 102 18 435 B4 ein Bewegungsmesssystem sowie ein paraokklusales Attachment zur Befestigung eines Bewegungssensors am Unterkiefer beschrieben. Das Attachment besteht aus einem Zahn-Befestigungsteil und einem Sensorik-Halteteil, an das Bewegungsmesssensoren bzw. -marker des Bewegungs-Analysesystems (etwa Ultraschall- oder optische Marker) angebracht werden können. Alternativ dazu kann die Sensorik des Bewegungsmesssystems direkt lösbar mit dem Zahn-Befestigungsteil verbunden sein. Bewegungsmesssensoren können beispielsweise Kamera- oder Lasersensoren, Magnetsensoren oder Sensoren basierend auf Beschleunigungsdetektoren oder Gyroskopen oder Kombinationen hieraus beinhalten. Zur Kompensation von Kopfbewegungen werden Referenz- oder Empfängersensoren am Oberkiefer oder über einen Kopfbogen befestigt. Weiterhin können stationäre Sensorsysteme z. B. Kameras erforderlich sein.

Ein einschlägiges Verfahren zur animierten bildlichen Darstellung von Bewegungen der Zähne des Unterkiefers für eine Erfassung der Bisslage und/oder von Bewegungsparametern anatomischer Punkte des Unterkiefers und/oder der Kieferrelation und/oder der Okklusion eines Wirbeltiers sowie ein entsprechendes System sind aus der JP 2020 501 830 A bekannt. Auch die DE 10 2018 26 097 A1 offenbart ein Verfahren und System mit dieser Zweckbestimmung, welches auf dem Einscannen der Zahnoberflächen einerseits und einer Positions- bzw. dynamischen Bewegungserfassung des Unterkiefers mit einer geeignet konstruierten und am Unterkiefer angebrachten Positionssensorik basiert.

Weiteren einschlägigen Stand der Technik bildet die CN 112 790 888 A.

Bei Verfahren der vorstehend beschriebenen Art wird grundsätzlich ein als Serienprodukt hergestelltes Attachment (nachfolgend auch bezeichnet als Positionssensor-Halteeinrichtung) am Unterkiefer des Patienten fixiert und mit diesem Attachment eine Erfassung von Positions- und Bewegungsdaten durchgeführt. In einem separaten Schritt wird ein Bild der Zahnoberflächen gewonnen, indem diese mittels eines intraoralen Scanners direkt eingescannt werden oder indem in traditioneller Weise mit einer mit Abdruckmasse gefüllten Bissgabel zunächst ein Abdruck der Zahnoberflächen erzeugt und dann (extraoral) dieser Abdruck eingescannt wird.

Die Erfindung ist durch die unabhängigen Ansprüche definiert.

Der Erfindung liegt die Aufgabe zu Grunde, ein verbessertes, insbesondere vereinfachtes und genaueres Verfahren der gattungsgemäßen Art sowie eine für ein solches Verfahren geeignete Positionssensor-Halteeinrichtung und ein entsprechendes System bereitzustellen.

Diese Aufgabe wird in ihrem Verfahrensaspekt mit einem Verfahren gemäß Anspruch 1 oder 2 und in ihrem Vorrichtungsaspekt mit einem System gemäß Anspruch 6 oder 7 gelöst. Zweckmäßige Fortbildungen des Erfindungsgedankens sind Gegenstand der jeweiligen abhängigen Ansprüche.

Die Erfindung schließt den Gedanken ein, das herkömmliche Verfahren durch den Einsatz einer Positionssensor-Halteeinrichtung zu vereinfachen, welche die unmittelbare und exakte Herstellung einer Positions-Relation zwischen den Zahnoberflächen des Patienten und der Positionssensorik erlaubt.

Dies ermöglicht den Verzicht auf eine zusätzliche Bissgabel bzw. es vereinfacht jedenfalls die mit dem Patienten durchzuführenden Abläufe. Die Befestigung der Messsensorik an den Zähnen ist mit einer solchen Halteeinrichtung schnell, einfach, mechanisch stabil und sehr präzise durchzuführen. Da die im vorgeschlagenen Verfahren zu benutzende Positionssensor-Halteeinrichtung in ihrem Aufbau inhärent eine Positions-Relation zwischen den Zahnoberflächen und der an der Halteeinrichtung befestigten Positionssensorik beinhaltet, entfallen arbeitsaufwändige Kalibrierschritte und werden Ungenauigkeiten vermieden, die bei der Verwendung von unspezifischen Serien-Halteeinrichtung und der eventuellen zusätzlichen Nutzung einer Bissgabel unvermeidlich sind. Erwähnenswert ist darüber hinaus die größere Bequemlichkeit des vorgeschlagenen Verfahrens für den Patienten und die sich hieraus ergebende größere Akzeptanz in der zahnärztlichen Praxis.

In einer Abwandlung des Verfahrens wird mit den durch das Einscannen gewonnenen Zahnoberflächendaten unmittelbar eine integrierte (einstückige) Halte-/Sensoreinrichtung konstruiert und hergestellt, welche eine Positionssensorik, also entsprechende Positionsmarkerelemente, integriert enthält. Hierbei entfällt das Zusammensetzen einer standardmäßig gefertigten Positionssensorik mit einer patientenindividuell gefertigten Positionssensor-Halteeinrichtung, was die Handhabung in der zahnärztlichen bzw. kieferchirurgischen Praxis vereinfacht.

Diese Abwandlung des erfindungsgemäßen Verfahrens schlägt sich auch darin nieder, dass im erfindungsgemäßen System anstelle einer Positionssensor-Halteeinrichtung und separaten Positionssensorik die erwähnte integrierte Halte-/Sensoreinrichtung konstruiert, hergestellt und bei den Positions- bzw. Bewegungsmessungen eingesetzt wird.

Es wird darauf hingewiesen, dass neben den erwähnten Zahnoberflächendaten optional auch Oberflächendaten des angrenzenden Zahnfleischs, welches zusammen mit den Zähnen gescannt wird, in dem Verfahren und System genutzt werden können.

Weiterhin wird darauf hingewiesen, dass die tatsächliche (optische) bildliche Darstellung bzw. die entsprechende Darstellungseinrichtung (Bildschirm) nicht bei jeder Anwendung des Verfahrens und Systems zwingend vorgesehen sein müssen. Die zu Grunde liegenden Bewegtbild-Daten können auch direkt in weitere Auswertungen bzw. diagnostische Schritte einfließen und wahlweise in einem solchen erweiterten Kontext bildlich dargestellt werden.

In einer praktisch bedeutsamen Ausführung des Verfahrens werden die durch das Scannen ermittelten Zahnoberflächendaten einer Recheneinheit einer CAD-Konstruktionsvorrichtung zugeführt, und die Positionssensor-Halteeinrichtung wird mittels eines CAD-Verfahrens konstruiert.

In weiteren praktischen bedeutsamen Ausführungen wird die Positionssensor-Halteeinrichtung unter Nutzung der durch das Scannen gewonnenen Zahnoberflächendaten aus einem vorgefertigten Kunststoffteil dreidimensional gefräst. Dementsprechend gehört zur Produktionseinrichtung in bevorzugten Ausführungen des Systems eine Fräsmaschine zum Fräsen mindestens der den Zähnen zugewandten Oberfläche der Positionssensor-Halteeinrichtung aus einem vorgefertigten Grundkörper, oder ein 3D-Drucker zum additiven Aufbau der Positionssensor-Halteeinrichtung insbesondere aus einem Polymermaterial.

Bei einer wichtigen Applikation des vorgeschlagenen Verfahrens handelt es sich um ein Verfahren zur Erfassung der Bisslage und/oder der Kieferrelation und Okklusion mit zusätzlicher Referenzmessung in definierter Position des Unterkiefers relativ zum Oberkiefer, insbesondere in habitueller Okklusion.

In dieser praktisch bedeutsamen Anwendung umfasst das Verfahren weiterhin die Schritte:
- Einscannen des Oberkiefers und animierte Darstellung der Bewegung der Zähne des Unterkiefers und Oberkiefers in Verbindung mit den Zahnoberflächendaten und Positionsrelationsdaten,
- Einscannen mindestens einer zusammenhängenden Zahnoberfläche des Oberkiefers und des Unterkiefers in einer definierten geschlossenen Kieferstellung zur Festlegung einer Startposition der animierten bildlichen Darstellung der Bewegung der Zähne.

Hierbei wird insbesondere die vordefinierte Position mit einem Registriermaterial gesichert.

In einer hierzu korrespondierenden Erweiterung des Systems weist das Positionsmesssystem eine mit der an den Zähnen befestigten Sensoreinrichtung zusammenwirkende Gesichtsbogen-Komponente, die an einem am Kopf des Wirbeltiers angebrachten Gesichtsbogen fixiert ist, oder eine raumfest angeordnete Komponente, auf.

Bezüglich des die Positionsmessung betreffenden Teils des vorgeschlagenen Verfahrens und Systems sowie auch bezüglich der vorstehend erwähnten Anwendung/Erweiterung wird darauf hingewiesen, dass diese dem Fachmann an sich bekannt sind, speziell auch aus Schutzrechtsveröffentlichungen der Anmelderin. Da auf dieses parate Wissen Bezug genommen werden kann, wird hier von einer vertieften Beschreibung dieser Schritte bzw. Komponenten und Ausführungen Abstand genommen.

Die bei dem Verfahren eingesetzte und eine zentrale Komponente des vorgeschlagenen Systems bildende Positionssensor-Halteeinrichtung umfasst
- einen in Anpassung an die Zahnreihe des Unterkiefers eines Wirbeltieres bogenförmig geformten Grundkörper, dessen eine Oberfläche zum Aufsetzen auf den Unterkiefer gemäß einem Abbild der Zahnoberflächen mindestens eines Abschnitts des Unterkiefers ausgebildet ist, und
- ein Sensoreinrichtungs-Halteelement, welches zur positions- und winkeltreuen Anbringung einer Positionssensorik an dem Grundkörper ausgebildet ist.

In einer Ausführung der Positionssensor-Halteeinrichtung umfasst diese hintere Stützabschnitte zur Abstützung im Bereich der hinteren Seitenzähne und wahlweise des darunterliegenden Zahnfleischs, wobei jeder hintere Stützabschnitt so geformt ist, dass er zumindest abschnittsweise in mesiale Richtung über oder unter einem hinteren Seitenzahn und über das angrenzende Zahnfleisch hinweg verläuft.

In einer weiteren Ausführung ist der Grundkörper mit dem Halteelement einstückig ausgebildet und insbesondere mittels eines Fräsverfahrens oder eines 3D-Druckverfahrens hergestellt. Es versteht sich, dass die zugehörige Positionssensorik ein an das Halteelement der Halteeinrichtung angepasstes Anschlussstück aufweist. Derartige Halteelement-/Anschlussstück-Kombinationen sind dem Fachmann gleichfalls aus dem Stand der Technik bekannt.

In einer weiteren Ausführung ist der Grundkörper mit dem Halteelement und einer Positionssensorik einstückig ausgebildet und im Wesentlichen mittels eines Fräsverfahrens oder eines 3D-Druckverfahrens hergestellt. Es wird hierbei vorausgesetzt, dass die Positionssensorik Merkmale enthält, die von einem Positionsmesssystem erkannt und verarbeitet werden können.

Auch ist es in einer Ausführung möglich, dass zu einer solchen gefrästen oder gedruckten Vorrichtung Komponenten einer Positionssensorik zugefügt werden, die abnehmbar sind oder nicht abnehmbar mit dem Grundkörper verbunden bleiben.

Nachfolgend wird die Erfindung auch hinsichtlich weiterer Merkmale und Vorteile anhand von Ausführungsbeispielen beschrieben, die anhand von Abbildungen näher erläutert werden. Hierbei zeigen:
Fig. 1 eine schematische Darstellung einer Ausführungsform des erfindungsgemäßen Verfahrens;
Fig. 2 eine schematische Darstellung einer Ausführungsform des erfindungsgemäßen Systems;
Fig. 3 eine Ausführungsform einer Positionssensor-Halteeinrichtung in einer schematischen Schrägansicht;
Fig. 4 eine erweiterte Ausführungsform gemäß Fig. 3 in einer Draufsicht;
Fig. 5 eine Ausführungsform mit einem Plateau in einer Ansicht von oben;
Fig. 6 die Ausführungsform gemäß Fig. 5 in einer Seitenansicht;
Fig. 7 eine weitere Ausführungsform des erfindungsgemäßen Attachments, in einer perspektivischen Ansicht;
Fig. 8 eine weitere Ausführungsform, in einer Seitenansicht und
Fig. 9 eine weitere Ausführungsform in einer perspektivischen Darstellung, zusammen mit einem Unterkiefer.

In der nachfolgenden Beschreibung werden für gleiche und gleich wirkende Teile weitgehend dieselben Bezugsziffern verwendet.

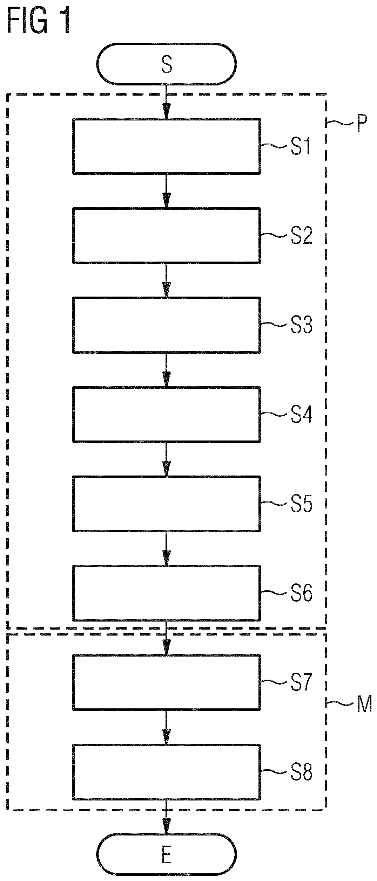

Fig. 1 zeigt eine schematische Darstellung eines Verfahrens zur Erfassung der Kieferrelation und/oder Okklusion.

In einem Schritt S1 werden zunächst Oberflächen von Zähnen des Unterkiefers, vorzugsweise mit einem Intraoral-Scanner erfasst und abgespeichert. Mittels dieser Zahnoberflächen-Daten wird in einem Schritt S2 eine Positionssensor-Halteeinrichtung, umfassend mindestens ein Halteelement zum Halten einer Positionssensorik (oder ein Halteelement und integrierter Positionssensorik) konstruiert. Die Halteeinrichtung wird an die erfassten Oberflächen angepasst, so dass es im auf den Oberkiefer aufgesetzten Zustand in vordefinierter Position und Orientierung gegenüber den Zähnen angeordnet ist und vorzugsweise das Gleiten der Zähne des Oberkiefers zu den Zähnen des Unterkiefers nicht behindert.

In einem Schritt S3, der ganz oder vollständig während des Schrittes S2 durchgeführt werden kann, oder im Anschluss an diesen, werden Positionsrelationsdaten zur Relativ-Position und Relativ-Orientierung einer mit der Halteeinrichtung verbundenen Positionssensorik gegenüber der an die gescannten Zahnoberflächen angepassten Oberfläche der Positionssensor-Halteeinrichtung abgespeichert.

In einem Schritt S4 wird das Attachment entweder mit einem abtragenden Bearbeitungsverfahren aus einem vorgefertigten Grundkörper herausgearbeitet oder mittels 3D-Druckes bzw. Additive Manufacturing schrittweise aus Polymer-Granulat oder einer Polymerpaste o. ä. aufgebaut. Danach wird in einem Schritt S5 eine Positionssensorik positions- und winkeltreu an dem Halteelement des Attachments fixiert oder vervollständigt (sofern diese nicht in den vorherigen Schritten bereits integriert wurde).

In einem Schritt S6 wird das Attachment mit daran fixierter Positionssensorik auf den Unterkiefer des Patienten aufgesetzt und hält dort allein aufgrund seiner präzisen Oberflächen-Passung, oder es wird zusätzlich mittels einer Haftcreme, und/oder einem z. B. Zweikomponenten oder lichthärtendem Klebematerial temporär fixiert.

Anschließend führt der Patient vorbestimmte Unterkieferbewegungen aus, und mittels der Positionssensorik, im Zusammenwirken mit einer Positionsmesseinrichtung (siehe weiter unten) werden in einem Schritt S7 Positions- bzw. Bewegungsdaten des Unterkiefers erfasst. Die Positions- bzw. Bewegungsdaten werden in Verbindung mit den o. g. Zahnoberflächendaten und Positionsrelationsdaten in einem Schritt S8 mittels eines herkömmlichen Simulationsprogrammes zu einer animierten Darstellung der Unterkieferbewegungen verarbeitet. Sie können im Weiteren u. a. für eine Bestimmung der Kieferrelation und sofern die Zahnflächen des Oberkiefers eingescannt wurden, zur Darstellung der Okklusion mit den Zähnen des Oberkiefers gemäß einem an sich bekannten Vorgehen genutzt werden.

In der Figur ist mit gestrichelten Linien symbolisiert, dass das Verfahren einen Konstruktions- und Herstellungsabschnitt P sowie einen Mess- und Auswertungsabschnitt M umfasst, die üblicherweise räumlich und zeitlich voneinander getrennt sind.

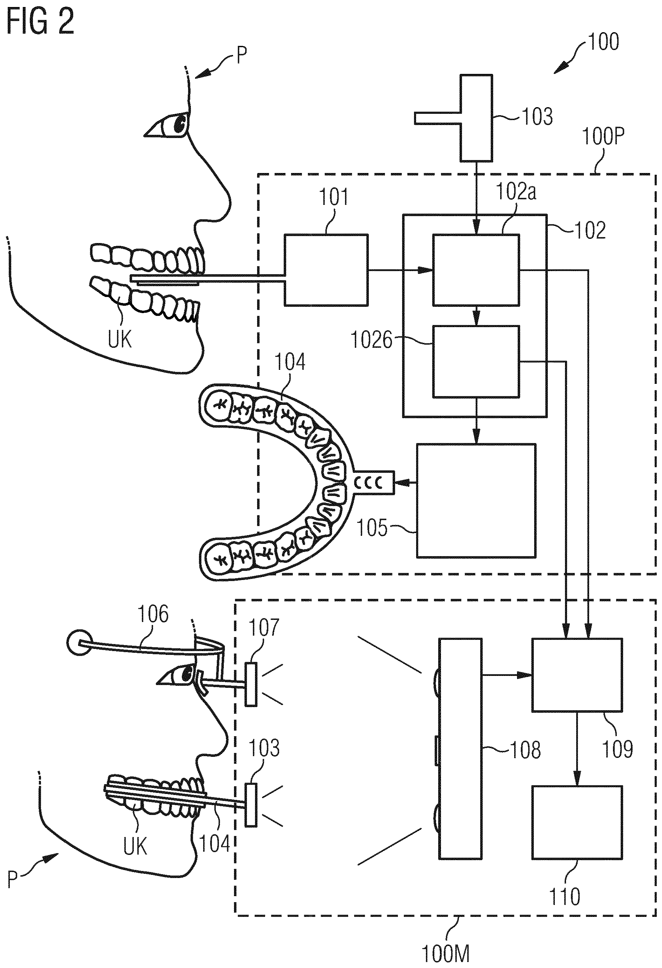

Dem entspricht auch der Aufbau einer entsprechenden Ausführungsform des erfindungsgemäßen Systems, wie er in Fig. 2 skizzenartig dargestellt ist.

Das Herstellungs- und Messsystem 100 umfasst einen Intraoralscanner 101, der schematisch im Einsatz zum Scannen der Unterkiefer(UK)-Zahnoberflächen eines Patienten P gezeigt ist. Der Intraoralscanner 101 ist, z.B. über eine Schnittstelle mit einer Datenspeichereinheit 102a eines CAD-Systems 102 zur Konstruktion einer erfindungsgemäßen Positionssensor-Halteeinrichtung verbunden, und die Zahnoberflächen-Daten werden dort abgelegt. Weiterhin werden in der Datenspeichereinheit 102a geometrische Daten einer hier mit der Ziffer 103 bezeichneten und symbolisch als Quader dargestellten Positionssensorik gespeichert, die zur positions- und winkeltreuen Fixierung an dem Attachment ausgebildet ist.

In einer Recheneinheit 102b des CAD-Systems 102 wird unter Nutzung der eingegebenen Daten eine patientenspezifische Positionssensor-Halteeinrichtung konstruiert, das hier mit Ziffer 104 bezeichnet ist. Abgebildet ist eine Ausführung mit abnehmbarer Positionssensorik. Parallel dazu wird ein Positionsrelations-Datensatz bereitgestellt, der aus den CAD-Daten der Positionssensor-Halteeinrichtung und den Konstruktionsdaten der Positionssensorik gebildet ist und die geometrische Relation zwischen der Positionssensoreinrichtung und den Zahnoberflächen der Unterkiefer-Zähne definiert, auf die das Attachment im Gebrauch aufgesetzt wird.

In einem 3D-Drucker 105 (oder einer Fräsmaschine) wird mit den aus der CAD-Einheit kommenden Konstruktionsdaten die Positionssensor-Halteeinrichtung hergestellt. Sofern nicht bereits integriert, wird anschließend die Positionssensorik 103 am Attachment fixiert, und das Attachment wird auf den Unterkiefer des Patienten aufgesetzt.

In der Figur ist dargestellt, dass der Patient außerdem einen Gesichtsbogen 106 mit einer weiteren Positionssensorik 107 trägt, die aber für die vorliegende Erfindung nicht wesentlich sind. Sie können jedoch bei einer auf das beanspruchte Verfahren folgenden Nutzung der Bewegungsdaten zur Bestimmung der Kieferrelation und/oder Okklusion (in an sich bekannter Weise) genutzt werden. Es sind Verfahren denkbar, bei denen auf die zusätzliche Sensorik 107 und/oder auf den Gesichtsbogen 106 verzichtet werden kann.

Im Zusammenwirken mit der Positionssensorik 103 erfasst ein raumfestes Positionsmesssystem 108 dynamisch die Positionsdaten des Unterkiefers und somit der Unterkiefer-Zahnoberflächen des Patienten während vorgegebener Bewegungen. Das raumfeste Positionsmesssystem 108 kann ersatzweise auch am Kopf getragen sein und in der Position des Sensors 107 mit der Positionssensorik 103 korrespondieren. Das Positionsmesssystem 108 ist ausgangsseitig mit einer Bewegtbild-Syntheseeinheit 109 verbunden, die aus den Bewegungsdaten, den CAD-Daten der Positionssensor-Halteeinrichtung 104 (entspr. den Zahnoberflächendaten) sowie den Positionsrelationsdaten eine animierte bildliche (virtuelle) Darstellung der Bewegung des Unterkiefers erzeugt. Diese wird auf einem Bildschirm 110 angezeigt; die Datensätze der virtuellen Bewegung können aber auch direkt in nachgeschaltete Auswertungs- bzw. Diagnoseschritte eingespeist werden.

Analog zur schematischen Darstellung des Verfahrens in Fig. 1 ist in Fig. 2 eine Unterteilung des Systems in eine Produktions-Sphäre 100P und eine Mess-Sphäre 100M mit gestrichelten Linien symbolisiert.

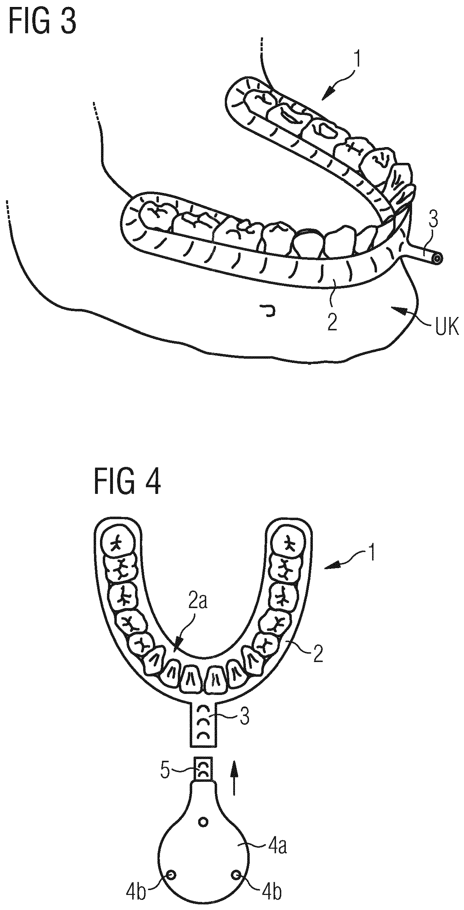

Fig. 3 zeigt eine beispielhafte Positionssensor-Halteeinrichtung 1, die einen bogenförmigen Grundkörper 2 und ein einstückig an diesen angeformtes Halteelement 3 aufweist, im am Unterkiefer UK eines Patienten aufgesetzten Zustand. Die Positionssensor-Halteeinrichtung kann durch eine 3D-Drucktechnik aus einem Polymer-Granulat oder einer Polymer-Paste aufgebaut sein und hat eine den Zähnen und dem Zahnfleisch des Unterkiefers zugewandte Oberfläche, die präzise an die entsprechenden Abschnitte der Zähne und des Zahnfleischs angepasst ist. Wie weiter oben erläutert, wird dies durch Einscannen der Zahnoberflächen und angrenzenden Zahnfleischoberflächen und Verwendung dieser Zahnoberflächendaten in einem CAD-Konstruktionsverfahren für die Positionssensor-Halteeinrichtung erreicht.

In der in Fig. 3 gezeigten Ausführung ist die Positionssensor-Halteeinrichtung derart geformt, dass sie die hinteren Seitenzähne des Unterkiefers vollständig umschließt, so dass die Halteeinrichtung in einem "Snap-Fit" auf die Unterkiefer-Zähne aufgesetzt werden kann und dort ggf. auch ohne Einsatz einer Haftcreme o. ä. in einer präzise vorgegebenen Position sitzt.

Fig. 4 zeigt in einer besonderen Ausführungsform die Positionssensor-Halteeinrichtung 1 zusammen mit einer Zwischenhaltevorrichtung 4a, die lösbar mit der Positionssensor-Halteeinrichtung 1 verbunden werden kann und die wiederum eine Haltevorrichtung für die Positionssensorik 4 enthält. Beispielhaft dargestellt ist hier eine Metallplatte mit drei Passmarken 4b, auf die eine mit Magneten versehene Positionssensorik 4 positionsgenau aufgesetzt werden kann.

Mittels eines Anschlussstücks 5 ist die Zwischenhaltevorrichtung 4a (oder alternativ direkt die Positionssensorik 4) exakt positions- und winkeltreu mit dem Halteelement 3 der Positionssensor-Halteeinrichtung 1 verbindbar. Das Halteelement 3 der Halteeinrichtung 1 und das Anschlussstück 5 der Positionssensoreinrichtung 4 können beispielsweise zueinander passende Gewindeabschnitte aufweisen oder insbesondere Rastmittel in Art eines Bajonettverschlusses. In der Figur ist auch das Zahnoberflächen-Profil 2a im Grundkörper 2 der Halteeinrichtung 1 gezeigt.

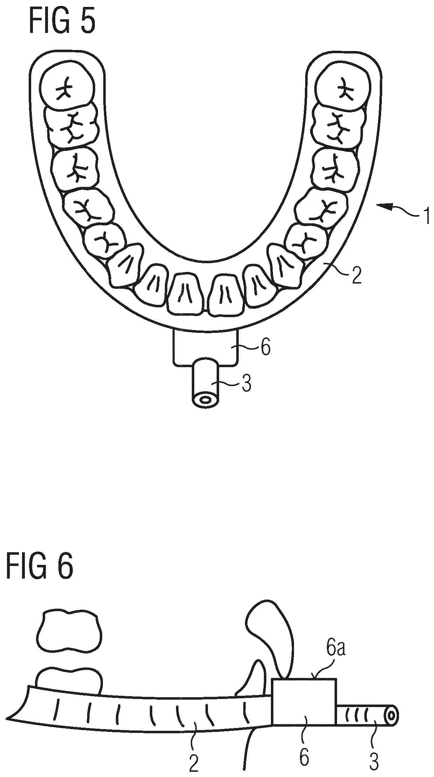

Fig. 5 und 6 zeigen in einer Draufsicht bzw. Seitenansicht eine modifizierte Positionssensor-Halteeinrichtung 1, bei der zwischen dem bogenförmigen Grundkörper 2 und dem Halteelement 3 zusätzlich ein Vorsprung 6 mit einem Plateau 6a angeformt ist. Auf das Plateau 6a können bei entsprechenden Kieferbewegungen die Frontzähne des Oberkiefers auftreffen, und es entkoppelt die Zähne des Oberkiefers gewissermaßen von denen des Unterkiefers und verhindert, insbesondere zur Bestimmung einer neuen Bissposition, die Okklusion. Die Höhe des Vorsprungs 6 kann vorzugsweise nach der Höhe einer künftigen Bisslage eines Aufbissbehelfs oder zahnärztlicher Restaurationen gewählt werden.

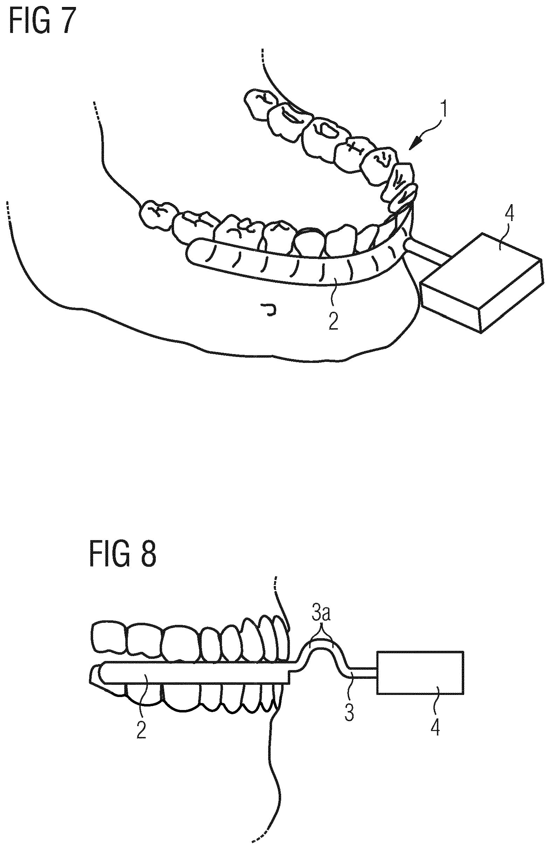

Fig. 7 zeigt - wiederum schematisch - eine weitere modifizierte Positionssensor-Halteeinrichtung 1, verbunden mit einer Positionssensorik 4, wobei hier der bogenförmige Grundkörper der Halteeinrichtung 1 sich nicht hinter den hinteren Seitenzähnen des Unterkiefers schließt, sondern lediglich einen vorderen Abschnitt des Unterkiefers umschließt. Hierbei kann eine zusätzliche Fixierung für den Bewegungs-/Messvorgang mittels Klebematerial sinnvoll sein, wodurch jedoch die präzise Positionsrelation zwischen der Sensoreinrichtung 4 und den Oberflächen der Unterkieferzähne nicht signifikant beeinträchtigt wird. Beispielhaft dargestellt ist hier, dass die Positionssensorik 4 als integraler Bestandteil der Positionssensor-Halteeinrichtung 1 gestaltet und nicht abnehmbar ist. Hierbei wird vorausgesetzt, dass die Positionssensorik 4 Messmittel bzw. Positionsmarker enthält, die von einem Positionsmesssystem erkannt werden können.

Fig. 8 zeigt eine weitere modifizierte Ausführung, bei der das Halteelement 3 eine Aufwölbung 3a nach oben einschließt. Hierdurch lässt sich die Halteeinrichtung angenehmer tragen, insbesondere bei der Durchführung von Kaubewegungen als vorbestimmte Bewegung, die vermessen werden soll.

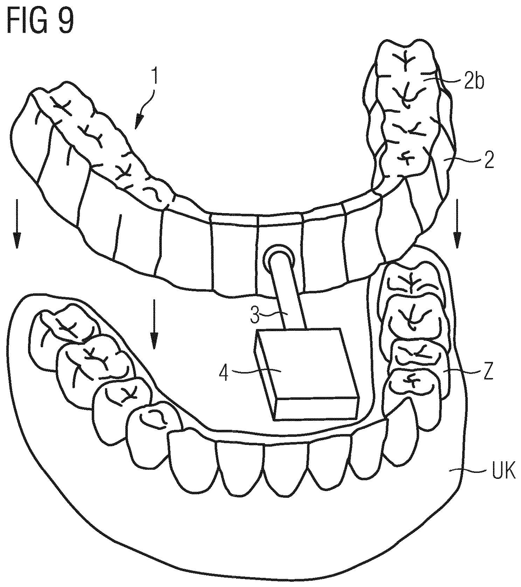

Während es sich bei den Positionssensor-Halteeinrichtungen nach Fig. 3 bis 8 um eine sog. paraokklusale Befestigung handelt, bei denen die Zähne von Unterkiefer und Oberkiefer in Okklusion gehen können und somit zahngeführte Bewegungen möglich sind, zeigt Fig. 9 eine okklusionsbedeckende Halteeinrichtung 1 mit angefügter Positionssensorik 4.

Die Okklusionsfläche 2b des bogenförmigen Grundkörpers 2 kann eine Zahn- oder wählbare andere Struktur beinhalten oder auch plan ausgeführt sein. Eine solche plane Fläche kann bei einer Positionsmessung bei geschlossenem Kiefer zur Definition einer Bisslage verwendet werden. Jedenfalls ist aber die den Zähnen Z des Unterkiefers UK zugewandte (nicht sichtbare) Oberfläche des Grundkörpers 2 aufgrund der Verwendung eines Zahnoberflächen-Scans bei der Konstruktion und Herstellung der Positionssensor-Halteeinrichtung 1 wiederum exakt an die Zahnoberflächen angepasst. Die starre Fixierung der Sensoreinrichtung 4 an der Halteeinrichtung 1 gewährleistet das Vorhandensein einer exakten Positionsrelation zwischen (nicht gezeigten) Markern der Sensoreinrichtung und den Zahnoberflächen der Zähne Z und ermöglicht somit eine direkte animierte Bilddarstellung der Zahnbewegungen ohne ein nochmaliges Scannen der Zahnoberflächen.

Bei einer bevorzugten Anwendung des vorgeschlagenen Verfahrens und Systems werden zusätzlich die Zähne des Oberkiefers und die des OK und des UK zusammen in Okklusion gescannt (Bukkal- bzw. Vestibular-Scan), sodass bei der virtuellen Darstellung eine definierte Ausgangsposition vorhanden ist. Diese "Kalibrierposition" kann in habitueller Okklusion oder durch ein eingebrachtes aushärtendes Material erzeugt werden. Die Startposition der Bewegungsmessung soll der jeweiligen Scan-Position entsprechen.

In einer besonderen Ausführungsform kann eine virtuelle Okklusionsanalyse auch mit einem okklusionsbedeckenden Attachment nach Fig. 9 durchgeführt werden, obwohl hier gar kein Zahnkontakt möglich ist. Dazu geht man wie folgt vor:
Es werden die Zähne des OK und UK gescannt. Danach werden die Punkte der Kiefergelenke z. B. mit einem Taststift in Relation zum Positionssensor und damit in Relation zum Attachment und den Zähnen bestimmt. Zur Okklusionsanalyse werden die Zähne des UK im hinteren Bereich anhand der Bewegungsspuren der beiden Kiefergelenke geführt, während sie im vorderen Bereich virtuell auf den Zahnflächen gleiten.

Der Vollständigkeit halber sei vermerkt, dass es neben dem Einsatz eines Taststiftes eine Reihe weiterer Möglichkeiten gibt, die Position beider Kondylenpositionen eines Patienten zu bestimmen. Man kann dazu in hinterer Kondylenposition eine Rotationbewegung des UK durchführen und die Drehpunkte bestimmen, oder man kann eine weite Öffnungsbewegung und eine Vorschubbewegung des UK durchführen und durch Iteration Punkte bestimmen, bei denen die Bewegungsspuren der Kondylenbahnen identisch sind. Des Weiteren gibt es die Möglichkeit, den Gesichtsbogen in Relation zum äußeren Gehörgang und damit in Relation zu den Kiefergelenken zu bringen.

Die zur Einbringung der Oberkieferlage in mechanische oder virtuelle Artikulatoren benötigte schädelbezügliche Kau- bzw. Okklusionsebene kann somit aus der bestimmten Position der Kondylen und aus einer gemittelten Ebene der Oberfläche des Zähne des Unterkiefers im geschlossenen Zustand und/oder der Ebene des Zusammentreffens der Oberfläche der Zähne des Unterkiefers mit dem Oberkiefer bestimmt werden.

## Patentansprüche

1. Verfahren zur animierten bildlichen Darstellung von Bewegungen der Zähne des Unterkiefers für eine Erfassung der Bisslage und/oder der Kieferrelation und/oder der Okklusion eines Wirbeltiers, aufweisend das Einscannen der Zahnoberflächen des Unterkiefers, insbesondere mit einem Intraoral- (101) oder Modellscanner, und Abspeichern der mit dem Scannen gewonnenen Zahnoberflächendaten,
nach den weiteren Schritten:
- Konstruieren einer Positionssensor-Halteeinrichtung (104), welche die Zähne des Unterkiefers mindestens zum überwiegenden Teil umschließt und die ein Halteelement zum Halten einer Positionssensorik (103) umfasst, unter Verwendung der Zahnoberflächendaten derart, dass eine den Unterkieferzähnen zugewandte Oberfläche der Halteeinrichtung exakt an die entsprechenden Zahnoberflächenabschnitte angepasst ist, so dass sie im Gebrauch in vordefinierter Position auf dem Unterkiefer sitzt,
- Herstellen der Positionssensor-Halteeinrichtung (104) nach den Konstruktionsdaten,
- Anbringen einer Positionssensorik (103) an der Positionssensor-Halteeinrichtung in vordefinierter Position und Winkellage,
- Abspeichern der aus Konstruktionsdaten gewonnenen Positionsrelation der Positionssensor-Halteeinrichtung zur Positionssensorik, die eine Positions-Relation zwischen der Positionssensorik und den gescannten Zahnoberflächen beschreibt,
- Einscannen mindestens einer zusammenhängenden Zahnoberfläche des Oberkiefers und des Unterkiefers in einer definierten geschlossenen Kieferstellung zur Festlegung einer Startposition der animierten bildlichen Darstellung der Bewegung der Zähne,
- Durchführen einer Positions- bzw. dynamischen Bewegungserfassung des mit der Positionssensorik versehenen Unterkiefers zur Gewinnung zeitabhängiger Positionsmessdaten,
- animierte bildliche Darstellung der Bewegung der Zähne des Unterkiefers aufgrund der Positionsmessdaten in Verbindung mit den Zahnoberflächendaten und Positionsrelationsdaten, und
- Einscannen der Zahnoberflächen des Oberkiefers und Referenzmessung in definierter Position des Unterkiefers relativ zum Oberkiefer, insbesondere in habitueller Okklusion.

2. Verfahren zur animierten bildlichen Darstellung von Bewegungen der Zähne des Unterkiefers für eine Erfassung der Bisslage und/oder der Kieferrelation und/oder der Okklusion eines Wirbeltiers, aufweisend das Einscannen der Zahnoberflächen des Unterkiefers, insbesondere mit einem Intraoral- (101) oder Modellscanner, und Abspeichern der mit dem Scannen gewonnenen Zahnoberflächendaten,
nach den weiteren Schritten:
- Konstruieren einer integrierten Halte-/Sensoreinrichtung, die einen Halteabschnitt, der Zähne des Unterkiefers mindestens zum überwiegenden Teil umschließt, und eine Positionssensorik (103) umfasst,
unter Verwendung der Zahnoberflächendaten derart, dass eine den Unterkieferzähnen zugewandte Oberfläche des Halteabschnitts exakt an die entsprechenden Zahnoberflächenabschnitte angepasst ist, so dass sie im Gebrauch in vordefinierter Position auf dem Unterkiefer sitzt,
- Herstellen der integrierten Halte-/Sensoreinrichtung nach den Konstruktionsdaten,
- Abspeichern der aus Konstruktionsdaten gewonnenen Positionsrelation der den Unterkieferzähnen zugewandten Oberfläche des Halteabschnitts zur Positionssensorik,
- Einscannen mindestens einer zusammenhängenden Zahnoberfläche des Oberkiefers und des Unterkiefers in einer definierten geschlossenen Kieferstellung zur Festlegung einer Startposition der animierten bildlichen Darstellung der Bewegung der Zähne,
- Durchführen einer Positions- bzw. dynamischen Bewegungserfassung des mit der Positionssensorik versehenen Unterkiefers zur Gewinnung zeitabhängiger Positionsmessdaten,
- animierte bildliche Darstellung der Bewegung der Zähne des Unterkiefers aufgrund der Positionsmessdaten in Verbindung mit den Zahnoberflächendaten und Positionsrelationsdaten, und
- Einscannen der Zahnoberflächen des Oberkiefers und Referenzmessung in definierter Position des Unterkiefers relativ zum Oberkiefer, insbesondere in habitueller Okklusion.

3. Verfahren nach Anspruch 1 oder 2, wobei die durch das Scannen ermittelten Zahnoberflächendaten einer Recheneinheit einer CAD-Konstruktionsvorrichtung zugeführt werden und die Positionssensor-Halteeinrichtung mittels eines CAD-Verfahrens konstruiert wird.

4. Verfahren nach einem der vorangehenden Ansprüche, wobei die Positionssensor-Halteeinrichtung oder integrierte Halte-/Sensoreinrichtung unter Nutzung der durch das Scannen gewonnenen Zahnoberflächendaten aus einem vorgefertigten Kunststoffteil dreidimensional gefräst oder mittels 3D-Druck insbesondere aus einem Polymermaterial aufgebaut wird.

5. Verfahren nach einem der vorangehenden Ansprüche, wobei die vordefinierte Position mit einem Registriermaterial gesichert und partielle Zahnoberflächen des Unter- und Oberkiefers zusammenhängend mit einem Intraoral- oder Modellscanner gescannt werden.

6. System zur animierten bildlichen Darstellung von Bewegungen der Zähne des Unterkiefers für eine Erfassung der Bisslage und/oder der Kieferrelation und/oder der Okklusion eines Wirbeltiers, zur Durchführung des Verfahrens nach einem der Ansprüche 1 und 3-5, wenn abhängig von Anspruch 1, aufweisend
- einen Intraoral- (101) oder Modellscanner zum Einscannen der Zahnoberflächen des Wirbeltieres, insbesondere Menschen, konfiguriert und betreibbar zum Einscannen mindestens einer zusammenhängenden Zahnoberfläche des Oberkiefers und des Unterkiefers in einer definierten geschlossenen Kieferstellung zur Festlegung einer Startposition der animierten bildlichen Darstellung der Bewegung der Zähne,
- eine Recheneinheit (102b) mit einer ersten Datenspeichereinrichtung (102a) einer CAD-Konstruktionsvorrichtung (102) zum Konstruieren einer Positionssensor-Halteeinrichtung (104) zur Anbringung einer Positionssensorik an den Zähnen des Unterkiefers des Wirbeltiers unter Benutzung der mit dem Intraoral-Scanner oder Modellscanner gewonnenen Zahnoberflächendaten,
- eine Produktionseinrichtung zur Herstellung der Positionssensor-Halteeinrichtung gemäß den durch die CAD-Konstruktionsvorrichtung gelieferten Konstruktionsdaten,
- eine Positionssensorik (103) zur Anbringung an der Positionssensor-Halteeinrichtung in vordefinierter Position und Winkellage,
- eine zweite Datenspeichereinrichtung zur Abspeicherung von aus Konstruktionsdaten der Positionssensorik und den Konstruktionsdaten der Positionssensor-Halteeinrichtung abgeleiteten Positionsrelationsdaten, die eine Positions-Relation zwischen der Positionssensorik und den gescannten Zahnoberflächen beschreiben,
- ein mit der Positionssensorik zusammenwirkendes Positionsmesssystem (108) zur Gewinnung von dynamischen Positions- bzw. Bewegungsdaten der Zähne, insbesondere des Unterkiefers,
- eine Bewegtbild-Darstellungseinrichtung (110) zur animierten bildlichen Darstellung der Bewegung der Zähne des Unterkiefers, die eingangsseitig mit dem Positionsmesssystem sowie der ersten und zweiten Datenspeichereinrichtung verbunden und zur Verknüpfung der Positionsmessdaten mit den Zahnoberflächendaten und den Positionsrelationsdaten ausgebildet ist, und
- eine Einrichtung zur Ausführung einer Referenzmessung in definierter Position des Unterkiefers relativ zum Oberkiefer, insbesondere in habitueller Okklusion, zur Darstellung der Bisslage und/oder der Kieferrelation und/oder der Okklusion.

7. System zur animierten bildlichen Darstellung von Bewegungen der Zähne des Unterkiefers für eine Erfassung der Bisslage des Unterkiefers und/oder der Kieferrelation und/oder der Okklusion eines Wirbeltiers, zur Durchführung des Verfahrens nach einem der Ansprüche 2 und 3-5, wenn abhängig von Anspruch 2, aufweisend
- einen Intraoral- (101) oder Modellscanner zum Einscannen der Zahnoberflächen des Wirbeltieres, insbesondere Menschen, konfiguriert und betreibbar zum Einscannen mindestens einer zusammenhängenden Zahnoberfläche des Oberkiefers und des Unterkiefers in einer definierten geschlossenen Kieferstellung zur Festlegung einer Startposition der animierten bildlichen Darstellung der Bewegung der Zähne,
- eine Recheneinheit (102b) mit einer ersten Datenspeichereinrichtung (102a) einer CAD-Konstruktionsvorrichtung (102) zum Konstruieren einer integrierten Halte-/Sensoreinrichtung zur Anbringung einer Positionssensorik (103) an den Zähnen des Unterkiefers des Wirbeltiers unter Benutzung der mit dem Intraoral-Scanner oder Modellscanner gewonnenen Zahnoberflächendaten,
- eine Produktionseinrichtung zur Herstellung der integrierten Halte-/Sensoreinrichtung gemäß den durch die CAD-Konstruktionsvorrichtung gelieferten Konstruktionsdaten,
- eine zweite Datenspeichereinrichtung zur Abspeicherung von aus Konstruktionsdaten der integrierten Halte-/Sensoreinrichtung abgeleiteten Positionsrelationsdaten, die eine Positions-Relation zwischen der Positionssensorik und den gescannten Zahnoberflächen beschreiben,
- ein mit der Positionssensorik : (103) zusammenwirkendes Positionsmesssystem (108) zur Gewinnung von dynamischen Positions- bzw. Bewegungsdaten der Zähne, insbesondere des Unterkiefers,
- eine Bewegtbild-Darstellungseinrichtung (110) zur animierten bildlichen Darstellung der Bewegung der Zähne des Unterkiefers, die eingangsseitig mit dem Positionsmesssystem sowie der ersten und zweiten Datenspeichereinrichtung verbunden und zur Verknüpfung der Positionsmessdaten mit den Zahnoberflächendaten und den Positionsrelationsdaten ausgebildet ist, und
- eine Einrichtung zur Ausführung einer Referenzmessung in definierter Position des Unterkiefers relativ zum Oberkiefer, insbesondere in habitueller Okklusion, zur Darstellung der Bisslage und/oder der Kieferrelation und/oder der Okklusion.

8. System nach Anspruch 6 oder 7, wobei die Produktionseinrichtung eine Fräsmaschine zum Fräsen mindestens der den Zähnen zugewandten Oberfläche der Positionssensor-Halteeinrichtung oder der integrierten Halte-/Sensoreinrichtung aus einem vorgefertigten Grundkörper oder einen 3D-Drucker zum additiven Aufbau der Positionssensor-Halteeinrichtung oder integrierten Halte-/Sensoreinrichtung insbesondere aus einem Polymermaterial aufweist.

9. System nach einem der Ansprüche 6 bis 7, wobei das Positionsmesssystem eine mit der an den Zähnen befestigten Sensorik zusammenwirkende Gesichtsbogen-Komponente, die an einem am Kopf des Wirbeltiers angebrachten Gesichtsbogen fixiert ist, oder eine raumfest angeordnete Komponente aufweist.

## Claims

1. Method for animated pictorial representation of movements of the teeth of the lower jaw for recording the bite position and/or the jaw relation and/or the occlusion of a vertebrate, comprising scanning the tooth surfaces of the lower jaw, in particular with an intraoral (101) or model scanner, and storing the tooth surface data obtained by scanning,
followed by the further steps of:
- constructing a position sensor holding device (104) which at least predominantly encloses the teeth of the lower jaw and which comprises a holding element for holding a position sensor (103), using the tooth surface data in such a way that a surface of the holding portion facing the lower jaw teeth is precisely adapted to the corresponding tooth surface portions so that, in use, it sits in a predefined position on the lower jaw,
- manufacturing the position sensor retaining device (104) according to the design data,
- attaching a position sensor system (103) to the position sensor holding device in a predefined position and angular position,
- storing the positional relationship of the position sensor holder to the position sensor obtained from the design data, which describes a positional relationship between the position sensor and the scanned tooth surfaces,
- scanning at least one contiguous tooth surface of the upper jaw and the lower jaw in a defined closed jaw position to determine a starting position for the animated visual representation of the movement of the teeth,
- performing a positional or dynamic motion detection of the lower jaw equipped with the position sensor system to obtain time-dependent position measurement data,
- animated pictorial representation of the movement of the teeth of the lower jaw based on the position measurement data in conjunction with the tooth surface data and position relation data, and
- scanning the tooth surfaces of the upper jaw and reference measurement in a defined position of the lower jaw relative to the upper jaw, in particular in habitual occlusion.

2. Method for animated visual representation of movements of the teeth of the lower jaw for recording the bite position and/or the jaw relation and/or the occlusion of a vertebrate, comprising scanning the tooth surfaces of the lower jaw, in particular with an intraoral (101) or model scanner, and storing the tooth surface data obtained by scanning,
followed by the further steps of:
- constructing an integrated holding/sensor device which has a holding section that at least substantially encloses the teeth of the lower jaw, and a position sensor system (103), using the tooth surface data in such a way that a surface of the holding portion facing the lower jaw teeth is precisely adapted to the corresponding tooth surface portions so that, in use, it sits in a predefined position on the lower jaw,
- manufacturing the integrated holding/sensing device according to the design data,
- storing the positional relationship of the surface of the retaining section facing the lower jaw teeth to the position sensor obtained from the design data,
- scanning at least one contiguous tooth surface of the upper jaw and lower jaw in a defined closed jaw position to determine a starting position for the animated visual representation of the movement of the teeth,
- performing positional or dynamic motion detection of the lower jaw equipped with the position sensor system to obtain time-dependent position measurement data,
- animated visual representation of the movement of the teeth of the lower jaw based on the position measurement data in conjunction with the tooth surface data and position relation data, and
- scanning the tooth surfaces of the upper jaw and reference measurement in a defined position of the lower jaw relative to the upper jaw, in particular in habitual occlusion.

3. Method according to claim 1 or 2, wherein the tooth surface data determined by scanning is fed to a computing unit of a CAD design device and the position sensor holding device is designed using a CAD method.

4. Method according to one of the preceding claims, wherein the position sensor holding device or integrated holding/sensor device is milled three-dimensionally from a prefabricated plastic part using the tooth surface data obtained by scanning, or is constructed by means of 3D printing, in particular from a polymer material.

5. Method according to one of the preceding claims, wherein the predefined position is secured with a registration material and partial tooth surfaces of the lower and upper jaw are scanned continuously with an intraoral or model scanner.

6. System for animated visual representation of movements of the teeth of the lower jaw for recording the bite position and/or the jaw relationship and/or the occlusion of a vertebrate, for carrying out the method according to one of claims 1 and 3-5, if dependent on claim 1, comprising
- an intraoral (101) or model scanner configured and operable for scanning the tooth surfaces of the vertebrate, in particular humans, for scanning at least one contiguous tooth surface of the upper jaw and the lower jaw in a defined closed jaw position for determining a starting position ( ) of the animated visual representation of the movement of the teeth,
- a computing unit (102b) with a first data storage device (102a) of a CAD design device (102) for designing a position sensor holding device (104) for attaching position sensors to the teeth of the lower jaw of the vertebrate using the tooth surface data obtained with the intraoral scanner or model scanner,
- a production device for manufacturing the position sensor holding device in accordance with the design data supplied by the CAD design device,
- a position sensor system (103) for attachment to the position sensor holding device in a predefined position and angular position,
- a second data storage device for storing positional relationship data derived from the design data of the position sensor and the design data of the position sensor holding device, which describe a positional relationship between the position sensor and the scanned tooth surfaces,
- a position measuring system (108) interacting with the position sensor system for obtaining dynamic position or movement data of the teeth, in particular of the lower jaw,
- a moving image display device (110) for animated visual representation of the movement of the teeth of the lower jaw, which is connected on the input side to the position measuring system and the first and second data storage devices and is designed to link the position measurement data with the tooth surface data and the position relation data, and
- a device for performing a reference measurement in a defined position of the lower jaw relative to the upper jaw, in particular in habitual occlusion, for displaying the bite position and/or the jaw relationship and/or the occlusion.

7. System for animated visual representation of movements of the teeth of the lower jaw for recording the bite position of the lower jaw and/or the jaw relation and/or the occlusion of a vertebrate, for performing the method according to one of claims 2 and 3-5, if dependent on claim 2, comprising
- an intraoral (101) or model scanner for scanning the tooth surfaces of the vertebrate, in particular humans, configured and operable for scanning at least one contiguous tooth surface of the upper jaw and the lower jaw in a defined closed jaw position to determine a starting position for the animated visual representation of the movement of the teeth,
- a computing unit (102b) with a first data storage device (102a) of a CAD design device (102) for designing an integrated holding/sensor device for attaching a position sensor (103) to the teeth of the lower jaw of the vertebrate using the tooth surface data obtained with the intraoral scanner or model scanner,
- a production device for manufacturing the integrated holding/sensing device in accordance with the design data supplied by the CAD design device,
- a second data storage device for storing positional relationship data derived from the design data of the integrated holding/sensing device, which describe a positional relationship between the position sensor and the scanned tooth surfaces,
- a position measuring system (108) cooperating with the position sensor system (103) for obtaining dynamic position or movement data of the teeth, in particular of the lower jaw,
- a moving image display device (110) for animated visual representation of the movement of the teeth of the lower jaw, which is connected on the input side to the position measuring system and the first and second data storage devices and is designed to link the position measurement data with the tooth surface data and the position relation data, and
- a device for performing a reference measurement in a defined position of the lower jaw relative to the upper jaw, in particular in habitual occlusion, for displaying the bite position and/or the jaw relationship and/or the occlusion.

8. System according to claim 6 or 7, wherein the production device comprises a milling machine for milling at least the surface of the position sensor holding device or the integrated holding/sensor device facing the teeth from a prefabricated base body, or a 3D printer for additively constructing the position sensor holding device or integrated holding/sensor device, in particular from a polymer material.

9. System according to one of claims 6 to 7, wherein the position measuring system comprises a facebow component cooperating with the sensor system attached to the teeth, which is fixed to a facebow attached to the head of the vertebrate, or a spatially fixed component.

## Revendications

1. Procédé de représentation graphique animée des mouvements des dents de la mâchoire inférieure pour enregistrer la position de la dentition et/ou la relation entre les mâchoires et/ou l'occlusion d'un vertébré, comprenant la numérisation des surfaces dentaires de la mâchoire inférieure, en particulier à l'aide d'un scanner intra-oral (101) ou d'un scanner de modèle, et l'enregistrement des données relatives à la surface dentaire obtenues lors de la numérisation,
suivi des étapes suivantes :
- la construction d'un dispositif de fixation de capteur de position (104) qui entoure au moins en grande partie les dents de la mâchoire inférieure et qui comprend un élément de fixation pour fixer un capteur de position (103), en utilisant les données relatives à la surface dentaire de telle sorte qu'une surface du dispositif de fixation tournée vers les dents de la mâchoire inférieure soit adaptée exactement aux sections correspondantes de la surface dentaire, de manière à ce qu'il repose en position prédéfinie sur la mâchoire inférieure lors de l'utilisation,
- fabrication du dispositif de fixation du capteur de position (104) selon les données de conception,
- fixation d'un capteur de position (103) sur le dispositif de fixation du capteur de position dans une position et une orientation angulaires prédéfinies,
- Enregistrement de la relation de position du dispositif de fixation du capteur de position par rapport au capteur de position, obtenue à partir des données de conception, qui décrit une relation de position entre le capteur de position et les surfaces dentaires scannées,
- numérisation d'au moins une surface dentaire contiguë de la mâchoire supérieure et de la mâchoire inférieure dans une position définie de la mâchoire fermée afin de déterminer une position de départ de la représentation graphique animée du mouvement des dents,
- réalisation d'un enregistrement de position ou de mouvement dynamique de la mâchoire inférieure équipée du capteur de position afin d'obtenir des données de mesure de position en fonction du temps,
- représentation graphique animée du mouvement des dents de la mâchoire inférieure sur la base des données de mesure de position en liaison avec les données de surface dentaire et les données de relation de position, et
- numérisation des surfaces dentaires de la mâchoire supérieure et mesure de référence dans une position définie de la mâchoire inférieure par rapport à la mâchoire supérieure, en particulier en occlusion habituelle.

2. Procédé de représentation graphique animée des mouvements des dents de la mâchoire inférieure pour enregistrer la position de l'occlusion et/ou la relation intermaxillaire et/ou l'occlusion d'un vertébré, comprenant la numérisation des surfaces dentaires de la mâchoire inférieure, en particulier à l'aide d'un scanner intra-oral (101) ou d'un scanner de modèle, et l'enregistrement des données de surface dentaire obtenues par numérisation,
suivies des étapes suivantes :
- la construction d'un dispositif de maintien/capteur intégré qui comprend une partie de maintien qui entoure au moins en grande partie les dents de la mâchoire inférieure, et un capteur de position (103), en utilisant les données relatives à la surface dentaire de telle sorte qu'une surface de la partie de maintien du dispositif de maintien tournée vers les dents de la mâchoire inférieure soit adaptée exactement aux parties correspondantes de la surface dentaire, de manière à ce qu'elle repose en position prédéfinie sur la mâchoire inférieure lors de l'utilisation,
- fabrication du dispositif de retenue/capteur intégré selon les données de conception,
- Enregistrement de la relation de position obtenue à partir des données de conception entre la surface de la partie de retenue tournée vers les dents de la mâchoire inférieure et le capteur de position,
- Numérisation d'au moins une surface dentaire contiguë de la mâchoire supérieure et de la mâchoire inférieure dans une position définie de la mâchoire fermée afin de déterminer une position de départ de la représentation graphique animée du mouvement des dents,
- réalisation d'un enregistrement de position ou de mouvement dynamique de la mâchoire inférieure équipée du capteur de position afin d'obtenir des données de mesure de position en fonction du temps,
- représentation graphique animée du mouvement des dents de la mâchoire inférieure sur la base des données de mesure de position en liaison avec les données de surface dentaire et les données de relation de position, et
- numérisation des surfaces dentaires de la mâchoire supérieure et mesure de référence dans une position définie de la mâchoire inférieure par rapport à la mâchoire supérieure, en particulier en occlusion habituelle.

3. Procédé selon la revendication 1 ou 2, dans lequel les données de surface dentaire déterminées par balayage sont transmises à une unité de calcul d'un dispositif de conception CAO et le dispositif de fixation du capteur de position est conçu à l'aide d'un procédé CAO.

4. Procédé selon l'une des revendications précédentes, dans lequel le dispositif de fixation du capteur de position ou le dispositif de fixation/capteur intégré est fraisé en trois dimensions à partir d'une pièce en plastique préfabriquée ou fabriqué par impression 3D, en particulier à partir d'un matériau polymère, en utilisant les données relatives à la surface dentaire obtenues par numérisation.

5. Procédé selon l'une des revendications précédentes, dans lequel la position prédéfinie est fixée à l'aide d'un matériau d'enregistrement et les surfaces dentaires partielles de la mâchoire inférieure et supérieure sont scannées de manière cohérente à l'aide d'un scanner intra-oral ou d'un scanner de modèle.

6. Système pour la représentation graphique animée des mouvements des dents de la mâchoire inférieure afin de déterminer la position de la dentition et/ou la relation entre les mâchoires et/ou l'occlusion d'un vertébré, pour la mise en œuvre du procédé selon l'une des revendications 1 et 3 à 5, si dépendant de la revendication 1, comprenant
- un scanner intra-oral (101) ou un scanner de modèle pour scanner les surfaces dentaires du vertébré, en particulier celles d'un être humain, configuré et pouvant fonctionner pour scanner au moins une surface dentaire continue de la mâchoire supérieure et de la mâchoire inférieure dans une position définie de la mâchoire fermée afin de déterminer une position de départ d' pour la représentation graphique animée du mouvement des dents,
- une unité de calcul (102b) avec un premier dispositif de stockage de données (102a) d'un dispositif de conception CAO (102) pour concevoir un dispositif de fixation de capteur de position (104) pour fixer un capteur de position sur les dents de la mâchoire inférieure du vertébré en utilisant les données de surface dentaire obtenues avec le scanner intra-oral ou le scanner de modèle,
- un dispositif de production pour la fabrication du dispositif de fixation du capteur de position conformément aux données de conception fournies par le dispositif de conception CAO,
- un capteur de position (103) destiné à être fixé au dispositif de fixation du capteur de position dans une position et un angle prédéfinis,
- un deuxième dispositif de stockage de données pour stocker des données de relation de position dérivées des données de conception du capteur de position et des données de conception du dispositif de fixation du capteur de position, qui décrivent une relation de position entre le capteur de position et les surfaces dentaires scannées,
- un système de mesure de position (108) coopérant avec le capteur de position pour obtenir des données de position ou de mouvement dynamiques des dents, en particulier de la mâchoire inférieure,
- un dispositif d'affichage d'images animées (110) pour la représentation graphique animée du mouvement des dents de la mâchoire inférieure, qui est relié en entrée au système de mesure de position ainsi qu'aux premier et deuxième dispositifs de stockage de données et qui est conçu pour relier les données de mesure de position aux données de surface dentaire et aux données de relation de position, et
- un dispositif pour effectuer une mesure de référence dans une position définie de la mâchoire inférieure par rapport à la mâchoire supérieure, en particulier en occlusion habituelle, pour représenter la position de l'occlusion et/ou la relation entre les mâchoires et/ou l'occlusion.

7. Système pour la représentation graphique animée des mouvements des dents de la mâchoire inférieure afin de déterminer la position occlusale de la mâchoire inférieure et/ou la relation maxillaire et/ou l'occlusion d'un vertébré, pour l' mise en œuvre du procédé selon l'une des revendications 2 et 3 à 5, si dépendant de la revendication 2, comprenant
- un scanner intra-oral (101) ou un scanner de modèle pour scanner les surfaces dentaires du vertébré, en particulier celles d'un être humain, configuré et pouvant fonctionner pour scanner au moins une surface dentaire contiguë de la mâchoire supérieure et de la mâchoire inférieure dans une position définie de la mâchoire fermée afin de déterminer une position de départ de la représentation graphique animée du mouvement des dents,
- une unité de calcul (102b) avec un premier dispositif de stockage de données (102a) d'un dispositif de conception CAO (102) pour concevoir un dispositif de fixation/capteur intégré pour la mise en place d'un système de capteurs de position (103) sur les dents de la mâchoire inférieure du vertébré à l'aide des données de surface dentaire obtenues avec le scanner intra-oral ou le scanner de modèle,
- un dispositif de production pour la fabrication du dispositif de fixation/capteur intégré conformément aux données de conception fournies par le dispositif de conception CAO,
- un deuxième dispositif de stockage de données pour enregistrer les données de relation de position dérivées des données de conception du dispositif de fixation/capteur intégré, qui décrivent une relation de position entre le capteur de position et les surfaces dentaires scannées,
- un système de mesure de position (108) coopérant avec le capteur de position (103) pour obtenir des données de position ou de mouvement dynamiques des dents, en particulier de la mâchoire inférieure,
- un dispositif d'affichage d'images animées (110) pour la représentation graphique animée du mouvement des dents de la mâchoire inférieure, qui est relié en amont au système de mesure de position ainsi qu'aux premier et deuxième dispositifs de stockage de données et qui est conçu pour relier les données de mesure de position aux données de surface dentaire et aux données de relation de position, et
- un dispositif pour effectuer une mesure de référence dans une position définie de la mâchoire inférieure par rapport à la mâchoire supérieure, en particulier en occlusion habituelle, afin de représenter la position de la dentition et/ou la relation entre les mâchoires et/ou l'occlusion.

8. Système selon la revendication 6 ou 7, dans lequel le dispositif de production comprend une fraiseuse pour fraiser au moins la surface tournée vers les dents du dispositif de fixation du capteur de position ou du dispositif de fixation/capteur intégré à partir d'un corps de base préfabriqué ou une
imprimante 3D pour la construction additive du dispositif de fixation du capteur de position ou du dispositif de fixation/capteur intégré, en particulier à partir d'un matériau polymère.

9. Système selon l'une des revendications 6 à 7, le système de mesure de position comportant un composant d'arc facial coopérant avec le système de capteurs fixé aux dents, qui est fixé à un arc facial monté sur la tête de l'animal vertébré, ou un composant disposé de manière fixe dans l'espace.
